# DEMANDE DE BREVET EUROPEEN

(11) **EP 0 826 368 A1**
(43) Date de publication de la demande: **04.03.1998**
(21) Numéro de dépôt: 97401998.6
(22) Date de dépôt: 27.08.1997
(51) Int. Cl.: A61K 31/19, A61K 31/195, A61K 31/12, A61K 31/165, A61K 31/445, A61K 31/24, A61K 31/63, A61K 31/38, A61K 31/215, A61K 31/34, A61K 31/235, A61K 31/455, A61K 31/60, A61K 31/20

(54) **Utilisation de rétinoides pour la préparation d'un médicament destiné à traiter les affections liées à une surexpression de VEGF**

(30) Priorité: 02.09.1996 FR 9610685
(71) Demandeur: CENTRE INTERNATIONAL DE RECHERCHES DERMATOLOGIQUES GALDERMA, ( CIRD GALDERMA), 06560 Valbonne (FR)
(72) Inventeur: Vega, Barbara, 06200 Nice (FR); Michel, Serge, 06330 Roquefort Les Pins (FR); Ladoux, Annie, 06000 Nice (FR); Frelin, Christian, 06100 Nice (FR)
(74) Mandataire: Tezier Herman, Béatrice

(57) **Abrégé**

La présente invention concerne l'utilisation de rétinoïdes, en particulier de rétinoïdes anti-AP-1 pour inhiber l'expression de VEGF. L'invention concerne également l'utilisation des dits rétinoïdes pour traiter les affections liées à une surexpression de VEGF, notamment le psoriasis, le syndrome de Kaposi, les dermatoses bulleuses, les tumeurs, etc.

## Description

La présente invention concerne l'utilisation de rétinoïdes, en particulier de rétinoïdes anti-AP-1 (Activator Protein-1) pour inhiber l'expression de VEGF. L'invention concerne également l'utilisation des dits rétinoïdes pour traiter les affections liées à une surexpression de VEGF, notamment le psoriasis, le syndrome de Kaposi, les dermatoses bulleuses, les tumeurs, les métastases, les angiomes, les rétinopathies diabétiques, le vieillissement photoinduit, les dermites d'irritation, l'eczéma, l'urticaire, l'hypersensibilité cutanée retardée, l'arthrite rhumatoïde ou l'allergie de contact.

VEGF, ou "Vascular Endothelial Growth Factor", est une glycoprotéine homodimérique de 34-36 kDa dont la séquence présente de l'ordre de 20 % d'identité avec les chaînes A et B du PDGF(TISCHER & al, J. Biol. Chem., 266, 1991, 11947-11954). La séquence d'ADN complémentaire de VEGF code pour un peptide signal qui lui permet d'être sécrété contrairement à FGF (Fibroblast Growth Factor) qui ne présente pas une telle séquence dans la structure de son gène. Les sites majeurs de production et les sites d'action étant différents, VEGF agit principalement sous un mode paracrine. La présence de VEGF a été retrouvée tant au niveau de nombreuses tumeurs que de tissus sains (SENGER D.L. & al, Cancer Metastasis Rev., 12, 1993, 303-324).

Dans la peau saine, l'expression de VEGF peut être augmentée à la suite d'une blessure (Brown L.F. & al, J. Exp. Med., 176, 1992, 1375-1379). La surexpression de VEGF a également été montrée dans de nombreuses affections de la peau associées à une hyperprolifération vasculaire et/ou à des modifications vasculaires, comme dans les plaques de psoriasis (DETMAR M. & al, J. Exp. Med., 180, 1994, 1141-1146), dans le syndrome de Kaposi (WEINDEL K. & al, Biochem. Biophys. Res. Commun., 183, 1992, 1167-74) ou encore dans les dermatoses bulleuses (BROWN L.F. & al, J. lnvest. Dermatol., 104, 1995, 744-749).

On sait, d'une manière générale, que l'acide rétinoïque tout-trans (all-trans retinoic acid) agit sur la différenciation et/ou la prolifération des cellules en interagissant avec des récepteurs nucléaires ou RARs (Retinoic Acid Receptors) contenus dans le noyau cellulaire. L'acide 9-cis rétinoïque interagit également avec les récepteurs RXRs (Récepteurs Rétinoïques X). De nombreux analogues synthétiques présentent une activité biologique analogue à celle de l'acide rétinoïque tout-trans ou de l'acide 9-cis-rétinoïque. Ils sont couramment dénommés "rétinoïdes". Parmi les rétinoïdes, on trouve plus particulièrement les agonistes RAR qui interagissent avec les récepteurs RARs. Il s'agit notamment de l'étrétinate ou de l'adapalène. ll existe, à ce jour, trois sous-types identifiés de récepteurs RAR appelés respectivement RAR-α, RAR-β et RAR-γ. Ces récepteurs, après fixation d'un ligand (i.e. l'acide rétinoïque tout-trans), interagissent avec la région promotrice de gènes régulés par l'acide rétinoïque au niveau d'éléments de réponses spécifiques (RARE).

Certains analogues peuvent se fixer et activer un sous-type de récepteur RAR (α, β ou γ) particulier. D'autres analogues, enfin, ne présentent aucune activité sélective particulière vis-à-vis de ces différents récepteurs. A cet égard, et par exemple, l'acide rétinoïque tout-trans active les RARs (ligand agoniste spécifique RARs), tous sous-types confondus.

De nombreux désordres et/ou affections dermatologiques peuvent être liés à une mauvaise régulation des récepteurs RARs. Ces désordres et/ou affections se traduisent le plus souvent par une composante inflammatoire, allergique et/ou immunologique. L'acide rétinoïque et les rétinoïdes comme l'étrétinate ou l'adapalène sont des médicaments employés pour le traitement de l'acné. L'acide rétinoïque a également été décrit pour le traitement thérapeutique des signes du vieillissement cutané, qu'il soit chronologique ou photoinduit (US 4 888 342).

Certains rétinoïdes ont été décrits comme capables de réguler l'expression de gènes comme les kératines (TOMIC-CANIC M. & al, J. Biol. Chem., 271, 1996, 1416-1423), ou de moduler l'activité du PDGF dans les fibroblastes psoriatiques (RAYNAUD F. & al, J. lnvest. Dermatol., 96, 1991, 111-115). Certains rétinoïdes ont également été décrits comme présentant une activité anti-AP-1, c'est à dire inhibant l'activité de AP-1 (WO 95/33745). A ce jour, aucun effet des rétinoïdes sur l'expression de VEGF n'a été rapporté dans la littérature (voir par exemple HARADA & al, J. Clin. lnvest., 93, 1993, 2490-2496).

On a maintenant pu montrer que les rétinoïdes, plus particulièrement les rétinoïdes présentant une activité anti-AP-1, inhibent l'expression de VEGF dans les kératinocytes.

La présente invention concerne donc l'utilisation d'au moins un rétinoïde présentant une activité anti-AP-1 pour inhiber l'expression de VEGF in vitro.

La présente invention concerne également l'utilisation d'au moins un rétinoïde présentant une activité anti-AP-1 pour la préparation d'un médicament destiné au traitement des affections liées à une surexpression de VEGF. Ces affections peuvent concerner différents organes ou tissus, et peuvent être notamment cutanées ou oculaires. Il s'agit en particulier du psoriasis, du syndrome de Kaposi, des dermatoses bulleuses, des tumeurs, des métastases, des angiomes, des rétinopathies diabétiques, du vieillissement photoinduit, des dermites d'irritation, de l'eczéma, de l'urticaire, de l'hypersensibilité cutanée retardée, de l'arthrite rhumatoïde ou de l'allergie de contact.

Enfin, la présente invention concerne une méthode de traitement des affections liées à une surexpression de VEGF, dans laquelle on administre une quantité appropriée de rétinoïde présentant une activité anti-AP-1, suffisante pour inhiber l'expression de VEGF dans le tissus ou l'organe affectés par une surexpression de VEGF.

Par rétinoïde, on entend selon l'invention tous les composés présentant un profil d'activité biologique analogue à celui de l'acide rétinoïque *tout-trans* ou de l'acide 9-cis-rétinoïque, ces composés peuvent moduler l'expression des gènes par le biais des récepteurs de la famille de l'acide rétinoïque, tels que les RARs et RXRs. Ainsi, les rétinoïdes selon l'invention peuvent donc présenter une activité dans le test de différenciation des cellules (F9) de tératocarcinome embryonnaire de souris (Cancer Research 43, p. 5268, 1983) et/ou dans le test d'inhibition de l'ornithine décarboxylase après induction par le TPA chez la souris (Cancer Research 38, p. 793-801, 1978). Ces tests montrent les activités de ces composés respectivement dans les domaines de la différenciation et de la prolifération cellulaire. Dans le test de différenciation des cellules (F9), il est possible d'évaluer une activité agoniste RARs, comme une activité antagoniste aux récepteurs de l'acide rétinoïque. En effet, un antagoniste est inactif lorsqu'il est seul dans ce test, mais inhibe partiellement ou totalement l'effet produit par un rétinoïde agoniste sur la morphologie et sur la sécrétion de l'activateur plasminogène. Ces composés peuvent donc présenter également une activité dans un test qui consiste à identifier des molécules antagonistes des RARs, tel que décrit dans la demande de brevet européen n° 0749755 déposée par la Demanderesse. D'autres rétinoïdes selon l'invention peuvent se lier aux récepteurs RXRs, certains possédant une activité agoniste, d'autres une activité antagoniste. Les propriétes de binding et de transactivation comme agoniste ou antagoniste aux récepteurs RXRs sont déterminées par des méthodes connues dans l'art, comme par exemple : MARTIN. B et all, Skin Pharmacol., 1992, **5**, 57-65 ; CAVEY. M. T. et al, Anal. Biochem., 1990, **186**, 19-23 ; LEVIN et al, Nature 1992, **355**, 359-61 ; ALLENBY et al, Proc. Natl. Acad. Sci., 1993, **90**, 30-4 ; ALLENBY et al, J. Biol. Chem., 1994, **269**, 16689-95.

De préférence, on utilise des rétinoïdes agonistes des RARs ou agonistes des RXRs. De manière encore plus préférentielle, on utilise les rétinoïdes agonistes des RARs.

Les rétinoïdes selon l'invention présentent également une activité anti-AP-1. Cette activité anti-AP-1 peut être mise en évidence selon les méthodes décrites dans la littérature, notamment la méthode décrite dans la demande de brevet WO 95/33745. L'activité anti-AP1 d'un composé peut donc être évaluée par des expériences de transactivation dans des cellules, telles les cellules Hela, en utilisant une partie du promoteur de la collagénase cloné en amont de la chloramphénicol actéyl transférase, soit sans co-transfection, en n'utilisant donc que les récepteurs endogènes, soit par co-transfection avec un récepteur d'expression codant pour des récepteurs RARs..

Parmi les rétinoïdes anti-AP-1, on peut citer les rétinoïdes décrits dans la demande de brevet WO 95/33745, ou encore ceux décrits notamment dans les demandes de brevets ou brevets EP 170 105, EP 199 636, EP 210 118, EP 210 929, EP 292 348, EP 409 728, EP 514 264, EP 514 269, EP 552 282, EP 568 898, EP 584 191, EP 658 553, EP 661 258, EP 679 630, EP 679 631, FR 2 677 020, FR 2 713 637, FR 2 713 635, FR 2 713 640, FR 2 719 044, FR 2 725 205, FR 2 726 274, FR 2 729 664, FR 2 731 706, FR 2 744 452, FR 2 746 101, FR 2 746 098 ou DE 28 19 213.

Parmi les rétinoïdes anti-AP-1, on peut notamment citer les composés suivants :
Composé 1 : acide 4-[(5,6,7,8-tétrahydro -5,5,8,8-tétraméthyl -2-naphtyl) carboxamido] benzoïque,
Composé 2 : acide 6-[3-(1-adamatyl) -4-méthoxyphényl] -2-naphtoïque,
Composé 3 : acide 6-[3-(1-adamatyl) -4-hydroxyphényl] -2-naphtoïque,
Composé 4: acide 4-(5,6,7,6-tétrahydro -5,5,8,8-tétraméthyl -2-anthracenyl) benzoïque,
Composé 5 : acide 4-[-1-hydroxy -(5,6,7,8-tétrahydro -5,5,8,8-tétraméthyl -2-naphtyl) -2-propynyl] benzoïque,
Acide 4-[2-(3-Adamantan-1-yl-4-methoxy-phenyl)-2-oxo-ethoxy]-2-hydroxy-benzoïque,
2-Hydroxy-4-[2-oxo-2-(5,5,8,8-tetramethyl-5,6,7,8-tetrahydro-naphtalen-2-yl)-ethoxy]-benzaldehyde,
2-(3-Hydroxy-4-methyl-phenoxy)-1-(5,5,8,8-tetramethyl-5,6,7,8-tetrahydro-naphtalen-2-yl)-ethanone,
N-Ethyl-2-hydroxy-4-[2-hydroxy-2-(5,5,8,8-tetramethyl-5,6,7,8-tetrahydro-naphtalen-2-yl)-ethoxy]-benzamide,
2-[3-Hydroxy-4-(piperidine-1-carbonyl)-phenoxy]-1-(5,5,8,8-tetramethyl-5,6,7,8-tetrahydro-naphtalen-2-yl)-ethanone,
3-tert-Butyl-4-methoxy-benzoic acid 4-(4-hydroxy-phenylcarbamoyl)-benzyl ester, Methyl 4-[2-hydroxy-2-(5,6,7,8-tetrahydro-5,5,8,8-tetramethyl-2-naphtyl)acetamido]phenyl sulfone,
Acide 5-[(E)-2-(3,5,5,8,8-pentamethyl-5,6,7,8-tetrahydro-naphtalen-2-yl)-propenyl]-thiophene-3-carboxylique,
Acide 4-(3,5,5,8,8-pentamethyl-5,6,7,8-tetrahydro-naphtalen-2-yloxy)-benzoïque,
Acide 6-(5,5,8,8-Tetramethyl-5,6,7,8-tetrahydro-anthracene-2-carbonyl)-naphtalene-2-carboxylique,
Acide 4-[(E)-2-(3-(1-methylcyclohexyl)-4-(6-tert-butoxycarbonylpentyloxy)phenyl) ethenyl]-benzoïque,
Méthyl ester de l'acide 4-(4,4-dimethyl-thiochroman-6-ylethynyl)-2-hydroxy-benzoïque,
Acide 2-Nonyloxy-4-(5,5,8,8-tetramethyl-5,6,7,8-tetrahydro-anthracen-2-yl)-benzoïque,
Acide 2-Hexyloxy-4-(5,5,8,8-tetramethyl-5,6,7,8-tetrahydro-anthracen-2-yl)-benzoïque,
Acide 3-[(5,6,7,8-tetrahydro-5,5,8,8-tetramethyl-2-naphtyl)(3-methyl)-2H-1-benzofuran]-6-carboxylique,
Acide 4-[6-methyloxycarbonyl-7-(1-adamantyl)-2-naphtyl]benzoïque,
Acide 4-[3-Adamantan-1-yl-4-(2-methoxy-ethoxymethoxy)-phenylethynyl]-2-hydroxy-benzoïque,
Acide 3-methyl-3-(1,2,3,4-tetrahydro-1,4a,9b-trimethyl-1,4-methanodibenzofuran-8-yl)-2H-1-benzofuran-6-carboxylique,
Acide 3-[(5,6,7,8-tetrahydro-5,5,8,8-tetramethyl-2-naphtyl)(3-allyl)-2H-1-benzofuran]-6-carboxylique,
Acide 2-Chloro-4-(3,5,5,8,8-pentamethyl-5,6,7,8-tetrahydro-naphtalen-2-ylsulfanyl)-benzoïque,
Acide 4-[6-(4-hydroxycarbonylbenzyloxy)-7-(1-adamantyl)-2-naphtyl]benzoïque,
Acide 6-(3-Adamantan-1-yl-4-hydroxy-phenyl)-naphtalene-2-carboxylique,
Acide 3-[3-(3,5,5,8,8-pentamethyl-5,6,7,8-tetrahydro-naphtalen-2-yl)-phenyl]-acrylique,
Acide 6-(3,5,5,8,8-pentamethyl-5,6,7,8-tetrahydro-naphtalen-2-ylulfanyl)-nicotinique,
Acide 4-[3-(5,6,7,8-tetrahydro-5,5,8,8-tetramethyl-2-naphtyl)-1-propynyl]salicylique,
Acide 4-(3,5,5,8,8-pentamethyl-5,6,7,8-tetrahydro-naphtalen-2-ylsulfanyl)-benzoïque,
Acide 2-Hydroxy-4-[3-hydroxy-3-(5,6,7,8-tetrahydro-5,5,8,8-tetramethyl-2-naphtyl)-1-propynyl]-benzoïque.

De préférence, on utilise les composés choisis parmi les composés 1 à 5 indiqués ci-dessus.

Les médicaments utiles selon l'invention peuvent se présenter sous forme de compositions pharmaceutiques, en particulier dermatologiques.

Les compositions pharmaceutiques comprenant une quantité appropriée d'au moins un rétinoïde présentant une activité anti-AP-1 comprend un support pharmaceutiquement acceptable et compatible avec le mode d'administration retenu.

La quantité appropriée dépendant bien entendu du traitement désiré et de la nature du composé choisi est donc déterminée par l'homme du métier.

L'administration des composés selon l'invention peut être effectuée par voie entérale, parentérale, topique ou oculaire.

Par voie entérale, la composition, plus particulièrement la composition pharmaceutique, peut se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, la composition, plus particulièrement la composition pharmaceutique, peut se présenter sous forme de solutions ou de suspensions pour perfusion ou pour injection.

Les composés selon l'invention sont généralement administrés à une dose journalière d'environ 0,01 mg/kg à 100 mg/kg en poids corporel, et ceci à raison de 1 à 3 prises.

Par voie topique, la composition est une composition dermatologique, plus particulièrement destinée au traitement de la peau et des muqueuses et peut alors se présenter sous forme d'onguents, de crèmes, de laits, de pommades, de poudres, de tampons imbibés, de solutions, de gels, de sprays, de lotions, de suspensions ou de shampooings. ll peut également se présenter sous forme de microsphères ou nanosphères ou vésicules lipidiques ou polymériques ou de patches polymériques et d'hydrogels permettant une libération contrôlée. Cette composition par voie topique peut par ailleurs se présenter soit sous forme anhydre, soit sous une forme aqueuse.

Par voie oculaire, ce sont principalement des collyres, des pommades ou des gels.

Cette composition à usage topique ou oculaire contient au moins un rétinoïde tel que défini ci-dessus à une concentration de préférence comprise entre 0,001% et 5% en poids par rapport au poids total de la composition.

La composition selon l'invention peut en outre contenir des additifs inertes ou même pharmacodynamiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des antibiotiques comme l'érythromycine et ses esters, la néomycine, la clindamycine et ses esters, les tétracyclines; des agents antifongiques tels que le kétoconazole ou les polyméthylène-4,5 isothiazolidones-3; des agents anti-inflammatoires non stéroïdiens; des caroténoïdes et, notamment, le β-carotène; des agents anti-psoriatiques tels que l'anthraline et ses dérivés; et enfin les acides eicosa-5,8,11,14-tétraynoïque et eicosa-5,8,11-triynoïque, leurs esters et amides.

La composition peut également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'a-tocophérol, le butylhydroxyanisole ou le butylhydroxytoluène.

L'inhibition de l'expresson de VEGF a été démontrée pour différents rétinoïdes par l'étude de l'expression des ARNm de VEGF dans les kératinocytes humains normaux en culture.

Le protocole expérimental et la méthode de détermination de l'expression de VEGF étaient les suivants:

### Culture des kératinocytes

Les cellules ont été isolées à partir de plasties mammaires et ont été ensemencées à partir d'un stock congelé au premier passage dans le milieu "Kératinocyte Basal Medium" (KBM, Promocell). Les facteurs de croissance suivants sont présents dans le milieu de culture: extrait pituitaire bovin 0,4 % (v/v), "epidermal growth factor" 10 ng/ml et insuline 5 µg/ml. Quand 80 % de la confluence est atteinte, le milieu de culture est changé et les composés dilués dans le DMSO sont ajoutés pendant 4 heures. Le contrôle est réalisé par addition de DMSO.

### Expression de VEGF

Les kératinocytes sont récupérés, puis les ARN totaux sont extraits. Un Northern blot est ensuite réalisé avec les différents échantillons d'ARN. La sonde spécifique VEGF utilisée est la séquence codante de la forme VEGF 165. La GAPDH (Glycéraldéhyde Phosphate Deshydrogénase) a été utilisée comme référence car son expression ne varie pas en fonction des différents traitements.

L'expression de VEGF a donc été calculée par le rapport VEGF/GAPDH.

Les résultats obtenus pour différents rétinoïdes sur l'expression de l'ARNm de VEGF dans les kératinocytes humains normaux (KHN) sont reportés dans le tableau ci-après.

| Composé | Concentration | Expression de VEGF |
|---|---|---|
| Contrôle | 10⁻⁶ M | 100 % |
| AR | 10⁻⁶ M | 70 % |
| 1 | 10⁻⁸ M | 60 % |
| 2 | 10⁻⁸ M | 58 % |
| 3 | 10⁻⁷ M | 66 % |
| 4 | 10⁻⁸ M | 76 % |
| AR = acide rétinoïque tout-trans | | |

L'activité du composé 5, rétinoïde anti-AP-1 spécifique, a été comparée à celle de la dexaméthasone, corticoïde utilisé dans le traitement du psoriasis, sur des KHN cultivés comme ci-dessus, le complexe AP-1 étant activé par l'addition de 10⁻⁷ M de TPA pendant 4 heures d'incubation.

| Composé | Concentration | Expression de VEGF |
|---|---|---|
| Contrôle (sans TPA) | - | 100 % |
| TPA | - | 260 % |
| TPA + composé 5 | 10⁻⁷ M | 169 % |
| TPA + dexaméthasone | 10⁻⁶ M | 120 % |

## Revendications

1. Utilisation d'au moins un rétinoïde présentant une activité anti-AP-1 pour inhiber l'expression de VEGF in vitro.

2. Utilisation d'au moins un rétinoïde présentant une activité anti-AP-1 pour la préparation d'un médicament destiné au traitement des affections liées à une surexpression de VEGF.

3. Utilisation selon la revendication 2, caractérisé en ce que le médicament est destiné au traitement des affections cutanées ou oculaires.

4. Utilisation selon l'une des revendications 2 ou 3, caractérisée en ce que le médicament est destiné au traitement du psoriasis, du syndrome de Kaposi, des dermatoses bulleuses, des tumeurs, des métastases, des angiomes, des rétinopathies diabétiques, du vieillissement photoinduit, des dermites d'irritation, de l'eczéma, de l'urticaire, de l'hypersensibilité cutanée retardée, de l'arthrite rhumatoïde ou de l'allergie de contact.

5. Utilisation selon l'une des revendications 2 à 4, caractérisée en ce que le médicament se présenter sous forme d'une composition pharmaceutique.

6. Utilisation selon la revendication 5, caractérisée en ce que la composition pharmaceutique est appropriée pour une administration par voie entérale, parentérale, topique ou oculaire.

7. Utilisation selon l'une des revendications 5 ou 6, caractérisée en ce que la composition est une composition dermatologique.

8. Utilisation selon la revendication 7, caractérisée en ce que la concentration concentration en rétinoïde est comprise entre 0,001% et 5% en poids par rapport au poids total de la composition.

9. Utilisation selon l'une des revendications 1 à 8, caractérisée en ce que les sont choisis parmi les composés suivants:
Acide 4-[(5,6,7,8-tétrahydro -5,5,8,8-tétraméthyl -2-naphtyl) carboxamido] benzoïque,
Acide 6-[3-(1-adamantyl)-4-méthoxyphényl]-2-naphtoïque,
Acide 6-[3-(1-adamantyl)-4-hydroxyphényl]-2-naphtoïque,
Acide 4-(5,6,7,8-tétrahydro -5,5,8,8-tétraméthyl -2-anthracenyl) benzoïque,
Acide 4-[-1-hydroxy -(5,6,7,8-tétrahydro -5,5,8,8-tétraméthyl -2-naphtyl) -2-propynyl] benzoïque,
Acide 4-[2-(3-Adamantan-1-yl-4-methoxy-phenyl)-2-oxo-ethoxy]-2-hydroxy-benzoïque,
2-Hydroxy-4-[2-oxo-2-(5,5,8,8-tetramethyl-5,6,7,8-tetrahydro-naphtalen-2-yl)-ethoxy]-benzaldehyde,
2-(3-Hydroxy-4-methyl-phenoxy)-1-(5,5,8,8-tetramethyl-5,6,7,8-tetrahydro-naphtalen-2-yl)-ethanone,
N-Ethyl-2-hydroxy-4-[2-hydroxy-2-(5,5,8,8-tetramethyl-5,6,7,8-tetrahydro-naphtalen-2-yl)-ethoxy]-benzamide,
2-[3-Hydroxy-4-(piperidine-1-carbonyl)-phenoxy]-1-(5,5,8,8-tetramethyl-5,6,7,8-tetrahyd ro-naphtalen-2-yl)-ethanone,
3-tert-Butyl-4-methoxy-benzoic acid 4-(4-hydroxy-phenylcarbamoyl)-benzyl ester,
Methyl 4-[2-hydroxy-2-(5,6,7,8-tetrahydro-5,5,8,8-tetramethyl-2-naphtyl)acetamido]phenyl sulfone,
Acide 5-[(E)-2-(3,5,5,8,8-pentamethyl-5,6,7,8-tetrahydro-naphtalen-2-yl)propenyl]-thiophene-3-carboxylique,
Acide 4-(3,5,5,8,8-pentamethyl-5,6,7,8-tetrahydro-naphtalen-2-yloxy)-benzoïque,
Acide 6-(5,5,8,8-Tetramethyl-5,6,7,8-tetrahydro-anthracene-2-carbonyl)-naphtalene-2-carboxylique,
Acide 4-[(E)-2-(3-(1-methylcyclohexyl)4-(6-tert-butoxycarbonylpentyloxy)phenyl) ethenyl]-benzoïque,
Méthyl ester de l'acide 4-(4,4-dimethyl-thiochroman-6-ylethynyl)-2-hydroxy-benzoïque,
Acide 2-Nonyloxy-4-(5,5,8,8-tetramethyl-5,6,7,8-tetrahydro-anthracen-2-yl)-benzoïque,
Acide 2-Hexyloxy-4-(5,5,8,8-tetramethyl-5,6,7,8-tetrahydro-anthracen-2-yl)-benzoïque,
Acide 3-[(5,6,7,8-tetrahydro-5,5,8,8-tetramethyl-2-naphtyl)(3-methyl)-2H-1-benzofuran]-6-carboxylique,
Acide 4-[6-methyloxycarbonyl-7-(1-adamantyl)-2-naphtyl]benzoïque,
Acide 4-[3-Adamantan-1-yl-4-(2-methoxy-ethoxymethoxy)-phenylethynyl]-2-hydroxy-benzoïque,
Acide 3-methyl-3-(1,2,3,4-tetrahydro-1,4a,9b-trimethyl-1,4-methanodibenzofuran-8-yl)-2H-1-benzofuran-6-carboxylique,
Acide 3-[(5,6,7,8-tetrahydro-5,5,8,8-tetramethyl-2-naphtyl)(3-allyl)-2H-1-benzofuran]-6-carboxylique,
Acide 2-Chloro-4-(3,5,5,8,8-pentamethyl-5,6,7,8-tetrahydro-naphtalen-2-ylsulfanyl)-benzoïque,
Acide 4-[6-(4-hydroxycarbonylbenzyloxy)-7-(1-adamantyl)-2-naphtyl]benzoïque,
Acide 6-(3-Adamantan-1-yl-4-hydroxy-phenyl)-naphtalene-2-carboxylique,
Acide 3-[3-(3,5,5,8,8-pentamethyl-5,6,7,8-tetrahydro-naphtalen-2-yl)-phenyl]-acrylique,
Acide 6-(3,5,5,8,8-pentamethyl-5,6,7,8-tetrahydro-naphtalen-2-ylulfanyl)-nicotinique,
Acide 4-[3-(5,6,7,8-tetrahydro-5,5,8,8-tetramethyl-2-naphtyl)-1-propynyl]salicylique,
Acide 4-(3,5,5,8,8-pentamethyl-5,6,7,8-tetrahyd ro-naphtalen-2-ylsulfanyl)benzoïque,
Acide 2-Hydroxy-4-[3-hydroxy-3-(5,6,7,8-tetrahydro-5,5,8,8-tetramethyl-2-naphtyl)-1-propynyl]-benzoïque.

10. Utilisation selon la revendication 9, caractérisée en ce que les composés sont choisis parmi les composés suivants:
Acide 4-[(5,6,7,8-tétrahydro -5,5,8,8-tétraméthyl -2-naphtyl) carboxamido] benzoïque,
Acide 6-[3-(1-adamantyl)-4-méthoxyphényl]-2-naphtoïque,
Acide 6-[3-(1-adamantyl) -4-hydroxyphényl] -2-naphtoïque,
Acide 4-(5,6,7,8-tétrahydro -5,5,8,8-tétraméthyl -2-anthracenyl) benzoïque,
Acide 4-[-1-hydroxy -(5,6,7,8-tétrahydro -5,5,8,8-tétraméthyl -2-naphtyl) -2-propynyl] benzoïque.
